Europäisches Patentamt

European Patent Office

Office européen des brevets

(11) Veröffentlichungsnummer: **0 332 086**

**A1**

(12) **EUROPÄISCHE PATENTANMELDUNG**

(21) Anmeldenummer: 89103794.7

(22) Anmeldetag: 03.03.89

(51) Int. Cl.⁴: **A47C 23/00 , A47C 27/00 , A61N 1/42**

(30) Priorität: 04.03.88 DE 3807131

(43) Veröffentlichungstag der Anmeldung:
13.09.89 Patentblatt 89/37

(84) Benannte Vertragsstaaten:
AT CH DE FR IT LI

(71) Anmelder: **Bohn, Gerhard**
**Wiesenstrasse 18**
**D-6433 Philippsthal(DE)**

(72) Erfinder: **Bohn, Gerhard**
**Wiesenstrasse 18**
**D-6433 Philippsthal(DE)**
Erfinder: **Bosner, Günter**
**Nehrener Strasse 4-6**
**D-7413 Gomaringen(DE)**
Erfinder: **Hauss, Reinard, Dr. rer. nat.**
**Kieler Strasse 71**
**D-2330 Eckernförde(DE)**

(74) Vertreter: **Wagner, Karl H.**
**WAGNER & GEYER Patentanwälte**
**Gewürzmühlstrasse 5 Postfach 246**
**D-8000 München 22(DE)**

(54) **Betteil.**

(57) Ein Betteil, insbesondere ein Ober- oder Unterbett, weist wenigstens eine von einem Strom durchflossene Erregerwicklung (2 bis 7) auf, die von einer
Stromquelle gespeist wird und ein magnetisches
Wechselfeld erzeugt. Das magnetische Wechselfeld
bzw. der Erregerstrom ist vorzugsweise sinus- oder
impulsförmig, wobei die Amplitude, Frequenz, das
Tastverhältnis und das Verhältnis der Zeiträume
ohne und mit Erzeugung eines magnetischen Wechselfelds einstellbar sind. Vorzugsweise sind an verschiedenen Stellen des Betteils für die jeweiligen
Körperteile Erregerwicklungen vorgesehen, deren
Erregerströme jeweils unabhängig voneinander bezüglich der besagten Parameter verän dert und eingestellt werden können.

Fig. 1

## Betteil

Die Erfindung betrifft ein Betteil, insbesondere ein Ober- oder Unterbett, mit wenigstens einem ein Magnetfeld erzeugenden Element.

Betteile dieser Art sind beispielsweise aus der EP-A3-0 144 610 desselben Anmelders bekannt. Bei diesem Bettteil ist ein magnetisches Element vorgesehen, das ein galvanisches Element aufweist, in dem magnetisierbare Teilchen enthalten sind. Letztere werden magnetisch so ausgerichtet, daß sich auf der einen Seite der magnetische Süd- und auf der anderen Seite der magnetische Nordpol bildet. Dadurch wird ein konstantes statisches Magnetfeld, beispielsweise im Zusammenhang mit einem Betteil gebildet. Statische Magnetfelder wurden zu Heilzwecken bereits im Altertum und im Mittelalter eingesetzt (vgl. z. B. W. Waldmann, Deutsches Arch. f. Geschichte d. Med. u. Med. Geographie, Leipzig, 1878, Seite 320 ff.). Auch in diesem Jahrhundert wurden statische Magnetfelder und deren positive Beeinflussung des menschlichen und tierischen Körpers in breitem Umfang untersucht. Der Beeinflussung biologischer Vorgänge durch magnetische Gleichfelder steht außer Frage.

In jüngster Zeit waren insbesondere auch magnetische Wechselfelder und deren Beeinflussung menschlicher und/oder tierischer Körper Gegenstand ausführlicher Untersuchungen. Im Zusammenhang mit der Applizierung von statischen Magnetfeldern, aber auch Magnet-Wechselfeldern, wurde insbesondere ein gerichtetes Zellwachstum bei der Wundheilung, ein gerichtetes Wachstum bei Pflanzenkernen, die Trennung von Leukozyten und und Erythrozyten, die Beeinflussung von Bakterienkulturen, sowie der Gleichgewichts- und Bewegungsvorgänge höherer und niederer Tiere festgestellt. Von besonderer Bedeutung sind Magnet-Wechselfelder zur positiven Beeinflussung von Heilerfolgen bei Knochenbrüchen. Die Bedeutung von Magnetfeldern in der Medizin steht außer Frage. Auf Grund der nachgewiesenen Heilerfolge von Therapiemethoden mit Magnetfeldern hat die kassenärztliche Bundesvereinigung Behandlungsverfahren mit Magnetfeldern etwa bei verzögerter Knochenbruch-Heilung, bei Pseudoarthrosen, Endoprothesenlockerungen, Hüftnekrosen und zahlreichen weiteren Leiden als Vertragsleistungen anerkannt.

Der Erfindung liegt die Aufgabe zugrunde, ein Betteil, insbesondere ein Ober- oder Unterbett zu schaffen, mit dem auf einfache Weise die Anwendung magnetischer Felder auf Körper oder Körperteile des Menschen, insbesondere im Schlafbereich, ermöglicht wird.

Ausgehend von dem eingangs genannten Betteil mit wenigstens einem ein Magnetfeld erzeugenden Element wird diese Aufgabe erfindungsgemäß dadurch gelöst, daß das Element eine von einem Strom durchflossene Erregerwicklung ist, die von einer Stromquelle gespeist wird und ein magnetisches Wechselfeld erzeugt.

Es konnte nämlich festgestellt werden, daß insbesondere Magnet-Wechselfelder im Schlafbereich das Wohlbefinden des Menschen nachhaltig verbessern können. Physiologische positive Auswirkungen von Magnet-Wechselfeldern im Schlafbereich ergaben sich insbesondere bei rheumatischen Beschwerden, Nervosität, Schlaflosigkeit und Streßsituationen.

Vorteilhafterweise ist das magnetische Wechselfeld ein im wesentlichen sinusförmiges Wechselfeld. Entsprechend wird die Erregerwicklung mit einem sinusförmigen Stromverlauf von der Stromquelle gespeist, was insbesondere auch schaltungstechnisch besonders einfach ist, da die Netz-Wechselfrequenz lediglich einer Frequenzänderung unterzogen werden muß und keine zusätzliche Signalformung erforderlich ist.

Vorteilhaft ist es dabei, daß die Frequenz des sinusförmigen Wechselfeldes in einem Bereich von 1 bis 14,5 Hz und vorzugsweise in einem Bereich von 3 bis 6 Hz liegt. Gerade derartige niedere Frequenzen in den genannten Bereichen wirken sich besonders günstig auf den menschlichen Organismus während der Schlafphase aus. Im unteren Frequenzbereich von 2 bis 6 Hz wirkt das sinusförmige Wechselfeld vornehmlich und für die meisten Menschen beruhigend und krampflösend, im Bereich von 6 bis 9 Hz stabilisierend und im Bereich von 10 bis 14,5 Hz schmerzstillend.

Im Hinblick auf eine digitalisierte Verarbeitung der Signale ist es alternativ auch möglich, daß das magnetische Wechselfeld ein im wesentlichen impulsförmiges Wechselfeld ist. In diesem Falle wird die Erregerwicklung von der Stromquelle mit einem impulsförmigen Strom gespeist. Auf Grund des Induktionsgesetzes wird in der Erregerwicklung durch den sich sprunghaft ändernden Strom ein entsprechendes Magnet-Wechselfeld induziert. Im Zusammenhang mit einem im wesentlichen impulsförmigen Wechselfeld ist es dabei wiederum vorteilhaft, wenn die Frequenz des impulsförmigen Wechselfeld in einem Bereich von 0,4 bis 14,5 Hz und vorzugsweise in einem Bereich von 3 bis 6 Hz liegt. Durch entsprechende Wahl der Frequenz des impulsförmiger Erregerstroms ergibt sich die gewünschte Frequenz des in der Erregerwicklung erzeugten impulsförmigen Wechselfelds.

Gemäß einer weiteren Ausführungsform der Erfindung ist die Frequenz des sinusförmigen bzw.

impulsförmigen Wechselfelds einstellbar. Dies wird durch Einstellen der Frequenz des Erregerstroms in der Erregerwicklung bewirkt.

Besonders vorteilhaft ist es, wenn das Tastverhältnis des impulsförmigen Wechselfelds in einem Bereich zwischen 0,2 bis 0,9 und vorzugsweise in einem Bereich von 0,3 bis 0,7 liegt. Vorteilhaft ist es dabei, wenn das Tastverhältnis einstellbar ist.

Gemäß einer vorteilhaften Ausgestaltung der Erfindung liegt die Feldstärke des magnetischen Wechselfeldes in einem Bereich von 100 bis 800 Gauss und vorzugsweise in einem Bereich von 100 bis 400 Gauss. Diese Bereiche sind Richtwerte.

Gemäß einer besonders vorteilhaften Ausführungsform der Erfindung ist vorgesehen, daß Zeiträume ohne Erzeugung eines magnetischen Wechselfelds mit Zeiten abwechseln, während denen ein magnetisches Wechselfeld erzeugt wird. Das bedeutet, daß der ruhende bzw. schlafende Körper nicht ständig, sondern in voneinander beabstandeten Zeitintervallen dem Magnet-Wechselfeld ausgesetzt wird. Es hat sich nämlich herausgestellt, daß eine derartige alternierende und nicht kontinuierliche Behandlung des ruhenden oder schlafenden Körpers mit einem Wechselfeld von besonderem Vorteil ist. Die Zeiträume ohne und mit Erzeugung eines magnetischen Wechselfelds können beispielsweise in der Größenordnung von Sekunden, aber auch von Minuten liegen. Vorteilhaft ist es dabei, wenn das Verhältnis der Zeiträume ohne und mit Erzeugung eines magnetischen Wechselfelds im wesentlichen 1:1 ist.

Eine weitere Ausführungsform der Erfindung besteht darin, das Verhältnis der Zeiträume ohne und mit Erzeugung eines magnetischen Wechselfelds je nach den vorliegenden Voraussetzungen und Erfordernissen bzw. je nach den individuellen Bedürfnissen eines Benutzers des erfindungsgemäßen Betteils einstellbar zu haben.

Eine besonders vorteilhafte Ausführungsform der Erfindung besteht darin, daß magnetische Erregerwicklungen an verschiedenen Stellen des Betteils und damit an verschiedenen Körperstellen des Benutzers vorgesehen sind. Auf diese Weise ist es individuell möglich, einzelne Stellen des Körpers gezielt und individuell einem magnetischen Wechselfeld auszusetzen. Dabei ist es vorteilhaft, daß insbesondere im Kopf-, Nacken-, Schulter-, Rücken-, Lenden-, Knie-, Knöchel- und/oder Fußbereich jeweils magnetische Erregerwicklungen vorgesehen sind. Die einzelnen Körperteile reagieren auf die Beaufschlagung von magnetischen Wechselfeldern unterschiedlich. Vorteilhaft ist es insbesondere auch, je nachdem, für welche Körperteile die Erregerwicklung vorgesehen ist, diese individuell hinsichtlich der Wicklungsform und Wicklungszahl auf die einzelnen Körperteile anzupassen, so daß dadurch insbesondere auch die Magnetfeldstärke und die Verteilung des Magnet-Wechselfelds den einzelnen Körperstellen angepaßt werden kann.

Besonders vorteilhaft ist es weiterhin, wenn die magnetischen Erregerwicklungen hinsichtlich der Frequenz, der Magnetfeldstärke, des Tastverhältnisses und/oder des Verhältnisses der Zeiträume ohne und mit Erregung des magnetischen Wechselfelds jeweils einzeln oder in Untergruppen zusammengefaßt einstellbar sind. Dies ermöglicht eine sehr individuelle, gesteuerte Applikation eines bestimmten, gewünschten magnetischen Wechselfelds jeweils individuell für einzelne Körperteile. Der Benutzer des erfindungsgemäßen Betteils kann dabei mittels Einstellknöpfen an einem Kontrollgerät die jeweiligen Parameter der einzelnen, für bestimmte Körperteile vorgesehenen Erregerwicklungen nach seinen Vorstellungen einstellen. Es ist auch möglich, Untergruppen für die Einstellung zusammenzufassen, beispielsweise Erregerwicklungen für den Schulter- und/oder Lendenbereich, wenn für diese Bereiche mehr als eine Erregerwicklung vorgesehen ist.

Vorteilhafterweise weist wenigstens einer der Erregerwicklungen einen Ferrit- oder Eisenkern auf. Der Ferrit- oder Eisenkern kann entsprechend geformt sein, derart, daß das Magnetfeld eine bestimmte, vorteilhafte Form mit optimaler Wirkung erhält.

Gemäß einer weiteren sehr vorteilhaften Ausführungsform der Erfindung sind die Erregerwicklungen in einer Zwischenschicht zwischen zwei Kunststoffschichten eines Unterbetts angeordnet. Auf diese Weise sind die Erregerwicklungen gegen äußere Einflüsse geschützt und auch die Verdrahtung der einzelnen Erregerwicklungen kann unsichtbar im Innern des Unterbetts erfolgen, so daß nur an einer Seite Leitungen austreten oder Anschlußbuchsen erforderlich sind. Die Kombination des erfindungsgemäßen Betteils mit der Verwendung an sich bekannter Schichten aus Aluminium, Kupfer, Aktivkohle und/oder einem elektrisch leitenden Kunststoff im Betteil ist besonders vorteilhaft. Auf diese Weise ergeben sich zusätzliche Wirkungen auf den Schläfer, die Additive, aber auch synergetische Effekte hervorrufen können. Die Vorteile und Ausführungsformen bei der Verwendung besagter Schichten sind beispielsweise in der deutschen Offenlegungsschrift 29 16 110, der europäischen Patentschrift 0 065 316 bzw. der deutschen Offenlegungsschrift 36 32 150 desselben Anmelders beschrieben, so daß darauf hier nicht weiter eingegangen zu werden braucht.

Besonders vorteilhaft ist es weiterhin, wenn dem magnetischen Wechselfeld ein magnetisches Gleichfeld überlagert ist. Auf diese Weise kann die Wirkung eines magnetischen Wechselfelds und eines magnetischen Gleichfelds kombiniert und ein noch größeres Wohlbefinden des Benutzers des erfindungsgemäßen Betteils erreicht werden.

Gemäß einer weiteren Ausgestaltung der Erfindung ist es insbesondere auch vorteilhaft, dem magnetischen Wechselfeld und/oder dem magnetischen Gleichfeld ein elektrostatisches Feld zu überlagern. Dadurch können zusätzliche positive Wirkungen auf die schlafende Person erzielt werden. Um Wiederholungen zu vermeiden, wird insbesondere auf die europäische Patentanmeldung 0 144 610 desselben Anmelders hingewiesen.

Die Erfindung wird nachstehend anhand der Zeichnungen beispielsweise erläutert. Es zeigen:

Fig. 1 eine schematische Darstellung des erfindungsgemäßen Betteils als Unterbett im Längsschnitt entlang der in Fig. 2 eingezeichneten Schnittlinie 1-1,

Fig. 2 eine schematische Darstellung eines Querschnitts des in Fig. 1 dargestellten Unterbetts entlang der in Fig. 1 eingezeichneten Schnittlinie 2-2,

Fig. 3 eine schematische Wiedergabe des magnetischen Wechselfeld- bzw. des Erregerstromverlaufs in Abhängigkeit der Zeit und

Fig. 4 ein weiteres Beispiel für den zeitlichen Verlauf des magnetischen Wechselfelds bzw. des das magnetische Wechselfeld erzeugenden Erregerstroms.

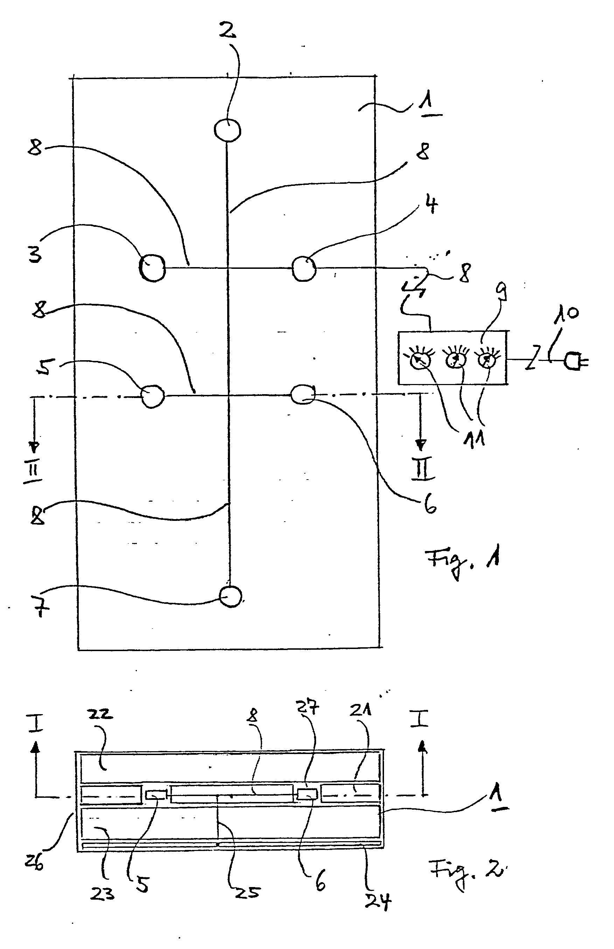

Fig. 1 zeigt ein Unterbett 1 im Längsschnitt, welches im Kopfbereich eine Erregerwicklung 2, im Schulterbereich Erreger wicklungen 3 und 4, im Lendenbereich Erregerwicklungen 5 und 6 und im Fußbereich eine Erregerwicklung 7 aufweist. Die Erregerwicklungen sind üblicherweise Spulen mit wenigstens einer Windung. Die Anzahl der Windungen sowie die Spulenform kann je nach den Erfordernissen und den gewünschten Parametern des zu erzeugenden magnetischen Wechselfelds gewählt werden.

Die Erregerwicklungen 2 bis 7 sind über schematisch dargestellte Leitungen 8 mit einer Bedienungseinrichtung 9 verbunden, die ihrerseits über eine Netzleitung 10 am Stromnetz angeschlossen werden kann. Die Bedienungseinrichtung 9 enthält eine Schaltungsanordnung, in der die Netzspannung auf eine Niedervolt-Spannung heruntertransformiert wird, und die wenigstens eine Stromformer-Schaltung aufweist, die die jeweiligen Erregerwicklungen 2 bis 7 mit Strom versorgt, derart, daß gewünschte Magnetfeld-Eigenschaften des in den Erregerwicklungen 2 bis 7 induzierten Magnet-Wechselfelds erzielt werden können. Wie im weiteren noch erläutert werden wird, können die Eigenschaften des in den Erregerwicklungen 2 bis 7 induzierten Magnet-Wechselfelds, etwa die Frequenz, die Magnetfeldstärke, das Tastverhältnis und/oder das Verhältnis der Zeiträume ohne und mit Erzeugung des magnetischen Wechselfelds dadurch eingestellt werden, daß die entsprechenden Parameter des Erregerstroms beispielsweise durch Bedienungsknöpfe 11 einer Bedienungseinrichtung 9 verändert werden.

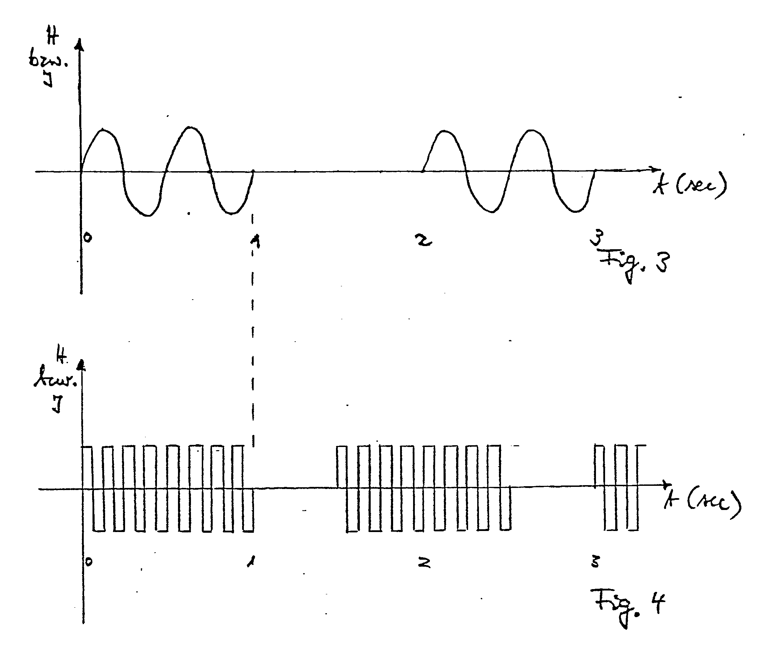

Die Leitungen 8, über die die Erregerwicklungen mit der Bedienungseinrichtung 9 und damit mit der (nicht dargestellten) Versorgungsschaltung verbunden sind, sind in Fig. 1 und 2 lediglich schematisch dargestellt. Selbstverständlich weisen die Erregerwicklungen 2 bis 7 jeweils zwei Anschlüsse auf, über die der Erregerstrom in die Erregerwicklung 2 bis 7 bzw. aus ihr heraus fließt.

Die einzelnen Erregerwicklungen 2 bis 7 können jeweils unab hängig voneinander mit der Bedienungseinrichtung verbunden undvon ihr mit dem Erregerstrom beaufschlagt werden. In diesem Falle ist es auch möglich, die besagten Magnetfeld-Parameter der einzelnen Erregerwicklungen 2 bis 7 jeweils getrennt voneinander mit unterschiedlichen, individuellen Erregerströmen zu versorgen. Auf diese Weise ist es möglich, dem der jeweiligen Erregerwicklung zugeordneten Körperteil einem diesem Körperteil spezifischen Magnet-Wechselfeld auszusetzen. Beispielsweise ist es daher möglich, den Kopf- und den Fußbereich des Schlafenden unterschiedlichen Magnet-Wechselfeldern auszusetzen, sei es hinsichtlich der Frequenz, der Magnetfeldstärke und/oder des Tastverhältnisses usw.

Es ist jedoch auch möglich, bestimmte Untergruppen von Erregerwicklungen, beispielsweise die im Schulterbereich angeordneten Erregerwicklungen 3 und 4 oder die im Lendenbereich angeordneten Erregerwicklungen 5 und 6 mit dem selben Erregerstrom zu beaufschlagen, etwa dadurch, daß die jeweiligen Erregerwicklungen in Reihe geschaltet sind. Selbstverständlich ist die Verwendung von Erregerwicklungen über die in Fig. 1 dargestellten Erregerwicklungen 2 bis 7 hinaus, etwa im Nacken- oder Kniebereich, möglich. Auch im Lenden- oder Schulterbereich können mehr als zwei Erregerwicklungen vorgesehen sein.

Der in Fig. 2 dargestellte schematische Querschnitt entlang der in Fig. 1 eingezeichneten Schnittlinie II-II zeigt die Erregerwicklungen 5 und 6 im Lendenbereich, die über die Leitung 8 miteinander und mit der Bedienungseinrichtung 9 verbunden sind. Die Erregerwicklungen 5 und 6 sind in einer Zwischenschicht 21 des Unterbettes angeordnet. Die Zwischenschicht 21 befindet sich beispielsweise zwischen zwei Schaumstoffauflagen 22 und 23. Bei dieser Ausführungsform des erfindungsgemäßen Unterbetts sind die Erregerwicklungen auf diese Weise geschützt zwischen den Schaumstoffauflagen 22 und 23 in der Zwischenschicht 21 angeordnet, so daß der auf dem Unterbett 1 liegende Schläfer von den eingearbeiteten Erregerwicklungen und den elektrischen Leitungen 8, mit denen die Erregerwicklungen mit der Bedienungseinrichtung 9 verbunden sind, nichts spürt.

Gemäß einer weiteren vorteilhaften Ausführungsform der Erfindung weist das Unterbett 1 eine elektrisch leitende Schicht 24, beispielsweise eine Aluminium- oder Kupferschicht auf, die auf eine Folie aufgebracht sein kann oder in Form von in das Gewebe eingearbeiteten Drähten oder Litzen vorliegt. Es ist auch möglich, als elektrisch leitende Schicht Aktivkohle oder einen elektrisch leitenden Kunststoff vorzusehen. Zur Ableitung statischer Aufladungen kann die elektrisch leitende Schicht 24 über eine Leitung 25 mit Masse verbunden sein, beispielsweise dadurch, daß die Masseleitung 25 zusammen mit den elektrischen Versorgungsleitungen 8 für die Erregerwicklungen zur Bedienungseinrichtung 9 geführt und dort mit Masse verbunden wird. Das gesamte Unterbett 1 ist mit einem Stoffüberzug 26 ummantelt. Die Schaumstoffauflagen 22 und 23 können eine unterschiedliche Dichte aufweisen. Beispielsweise kann die obere Schaumstoffauflage 22 eine Dichte von 45 kg/m³ und die untere Schaumstoffauflage 23 eine Dichte von 30 kg/m³ haben. Auf diese Weise ist es möglich, lediglich durch Umdrehen des Unterbetts 1 ein unterschiedlich hartes Bett zu erhalten. Die mit Ausnehmungen 27 für die Erregerwicklungen 2 bis 7 ausgebildete Zwischenschicht 21 weist beispielsweise eine gegenüber den Schaumstoffauflagen 22 und 23 geringere Dichte von 20 kg/m³ auf.

Fig. 3 zeigt ein Beispiel für den zeitlichen Verlauf eines sinusförmigen Magnet-Wechselfelds bzw. des entsprechenden Erregerstroms, der durch die Erregerwicklungen 2 bis 7 fließt. In dem in Fig. 3 dargestellten Ausführungsbeispiel beträgt die Frequenz des sinusförmigen Wechselfelds bzw. des sinusförmigen Erregerstroms 2 Hz, also zwei Schwingungen pro Sekunde. Nach einer Sekunde tritt ein Zeitraum ohne Erzeugung eines Wechselfelds auf, während nach 2 Sekunden wiederum eine eine Sekunde dauernde Erzeugung eines Wechselfelds vorliegt. Bei der in Fig. 3 dargestellten Ausführungsform wechseln also Zeiträume ohne und mit Erzeugung eines magnetischen Wechselfelds ab, wobei das Verhältnis dieser Zeiträume bei diesem Beispiel 1:1 ist.

In Fig. 4 ist ein Beispiel für ein impulsförmiges Wechselfeld bzw. für ein Wechselfeld dargestellt, das mittels impulsförmigen Erregerströmen erzeugt wird. Wie aus Fig. 4 ersichtlich ist, treten pro Sekunde acht Impulszyklen auf, so daß die Impulsfrequenz 8 Hz beträgt. Daran schließt sich ein Zeitraum ohne Erzeugung eines magnetischen Wechselfelds an. Dieser Zeitraum beträgt 0,5 Sekunden. Auf diesen Zeitraum ohne Erzeugung eines magnetischen Wechselfelds folgt ein Zeitraum von einer Sekunde, während dem das magnetische Wechselfeld wieder erzeugt wird. Die Intervalle ohne und mit Erzeugung eines magnetischen Wechselfelds wiederholen sich in der beschriebenen Weise. Das Verhältnis der Zeiträume ohne und mit Erzeugung eines magnetischen Wechselfelds ist bei dieser Ausführungsform 1:2, so daß das magnetische Wechselfeld insgesamt über einen längeren Zeitraum auf den Schleifer einwirkt als bei dem anhand von Fig. 3 beschriebenen Beispiel. Das Tastverhältnis des impulsförmigen Wechselfelds gemäß Fig. 4 ist 0,5.

Bei einer bevorzugten Ausführungsform der Erfindung ist die Amplitude und/oder die Frequenz und/oder das Tastverhältnis des Erregerstroms und/oder das Verhältnis der Zeiträume ohne und mit Erregerstrom einstellbar. Auf diese Weise ist es möglich, ein dem individuellen Benutzer und den jeweiligen Umständen angepaßtes Magnet-Wechselfeld im Schlafbereich zu erzeugen. Insbesondere ist es, wie dies bereits angedeutet wurde, auch möglich, für die einzelnen Erregerwicklungen die genannten Parameter unabhängig von den anderen Erregerwicklungen vom Benutzer je nach Wunsch und den gegebenen Erfordernissen einzustellen und zu ändern.

Die Erfindung wurde anhand bevorzugter Ausführungsformen beschrieben. Dem Fachmann sind jedoch zahlreiche Abwandlungen, Ausgestaltungen und Ergänzungen möglich, ohne daß dadurch der Erfindungsgedanke verlassen wird. Beispielsweise kann ein magnetisches Wechselfeld mit einem magnetischen Gleichfeld, einem elektrischen Wechselfeld und/oder einem elektrischen Gleichfeld überlagert werden, so daß verschiedene Effekte additiv oder mit synergetischer Wirkung auftreten, die das Wohlbefinden des Schläfers noch weiter verbessern.

Die erfindungsgemäßen Merkmale sind insbesondere nicht auf den Schlaf- und Bettenbereich beschränkt. Vielmehr können diese Merkmale auch im Zusammenhang mit anderen Anwendungsformen verwirklicht werden. Beispielsweise ist es möglich, Kleidungsartikel mit Erregerwicklungen an geeigneten oder bevorzugten Stellen des Körpers vorzusehen, wobei die Bedienungseinrichtung mit der Schaltungsanordnung zum Versorgen der Erregerwicklungen mit Erregerstrom mit im Kleidungsstück oder in einer Tasche desselben integriert ist. Die Energieversorgung einer derartigen Anordnung erfolgt dabei vorzugsweise mit einer Batterie.

## Ansprüche

1. Betteil, insbesondere Ober- oder Unterbett, mit wenigstens einem ein Magnetfeld erzeugenden Element, dadurch **gekennzeichnet**, daß das Element eine von einem Strom durchflossene Erregerwicklung (2 bis 7) ist, die von einer Stromquelle gespeist wird und ein magnetisches Wechselfeld erzeugt.

2. Betteil nach Anspruch 1, dadurch gekennzeichnet, daß das magnetische Wechselfeld ein im wesentlichen sinusförmiges Wechselfeld ist.

3. Betteil nach Anspruch 2, dadurch gekennzeichnet, daß die Frequenz des sinusförmigen Wechselfelds in einem Bereich von 0,4 bis 14,5 Hz und vorzugsweise in einem Bereich von 3 bis 6 Hz, liegt.

4. Betteil nach Anspruch 1, dadurch gekennzeichnet, daß das magnetische Wechselfeld ein im wesentlichen impulsförmiges Wechselfeld ist.

5. Betteil nach Anspruch 4, dadurch gekennzeichnet, daß die Frequenz des impulsförmigen Wechselfelds in einem Bereich von 0,4 bis 14,5 Hz und vorzugsweise in einem Bereich von 3 bis 6 Hz, liegt.

6. Betteil nach Anspruch 5, dadurch gekennzeichnet, daß das Tastverhältnis des impulsförmigen Wechselfelds in einem Bereich von 0,2 bis 0,9 und vorzugsweise in einem Bereich von 0,3 bis 0,7 liegt.

7. Betteil nach einem der Ansprüche 1 bis 6, dadurch gekenn zeichnet, daß die Frequenz des magnetischen Wechselfelds einstellbar ist.

8. Betteil nach einem der Ansprüche 1 bis 7, dadurch gekennzeichnet, daß das Tastverhältnis einstellbar ist.

9. Betteil nach einem der Ansprüche 1 bis 8, dadurch gekennzeichnet, daß die Feldstärke des magnetischen Wechselfelds in einem Bereich von 100 bis 800 Gauss und vorzugsweise in einem Bereich von 100 bis 400 Gauss liegt.

10. Betteil nach einem der Ansprüche 1 bis 9, dadurch gekennzeichnet, daß die Zeiträume ohne Erzeugung eines magnetischen Wechselfelds mit Zeiträumen abwechseln, während denen ein magnetisches Wechselfeld erzeugt wird.

11. Betteil nach Anspruch 10, dadurch gekennzeichnet, daß das Verhältnis der Zeiträume ohne und mit Erzeugung eines magnetischen Wechselfelds im wesentlichen 1:1 ist.

12. Betteil nach einem der Ansprüche 1 bis 11, dadurch gekennzeichnet, daß das Verhältnis der Zeiträume ohne und mit Erzeugung eines magnetischen Wechselfelds einstellbar ist.

13. Betteil nach einem der Ansprüche 1 bis 12, dadurch gekennzeichnet, daß magnetische Erregerwicklungen (2 bis 7) an verschiedenen Stellen des Betteils (1) vorgesehen sind.

14. Betteil nach Anspruch 13, dadurch gekennzeichnet, daß magnetische Erregerwicklungen (2 bis 7) im Kopf-, Nacken-, Schulter-, Rücken-, Lenden-, Knie-, Knöchel- und/oder Fußbereich vorgesehen sind.

15. Betteil nach Anspruch 13 oder 14, dadurch gekennzeichnet, daß die magnetischen Erregerwicklungen (2 bis 7) hinsichtlich der Frequenz, der Feldstärke, des Tastverhältnisses und/oder des Verhältnisses der Zeiträume ohne und mit Erzeugung des magnetischen Wechselfelds jeweils einzeln oder in Untergruppen zusammengefaßt einstellbar sind.

16. Betteil nach einem der Ansprüche 1 bis 15, dadurch gekennzeichnet, daß wenigstens eine der Erregerwicklungen (2 bis 7) einen Ferrit- oder Eisenkern aufweist.

17. Betteil nach einem der Ansprüche 1 bis 16, dadurch gekennzeichnet, daß die Erregerwicklungen (2 bis 7) in einer Zwischenschicht (21) zwischen zwei Schaumstoffschichten (22, 23) eines Unterbetts (1) angeordnet sind.

18. Betteil nach einem der Ansprüche 1 bis 17, dadurch gekennzeichnet, daß eine Schicht aus Aluminium, Kupfer, Aktivkohle und/oder einem elektrisch leitenden Kunststoff vorgesehen ist.

19. Betteil nach Anspruch 18, dadurch gekennzeichnet, daß die Schicht geerdet ist.

20. Betteil nach einem der Ansprüche 1 bis 19, dadurch gekennzeichnet, daß dem magnetischen Wechselfeld ein magnetisches Gleichfeld überlagert ist.

21. Betteil nach einem der Ansprüche 1 bis 20, dadurch gekennzeichnet, daß dem magnetischen Wechselfeld und/oder dem magnetischen Gleichfeld ein elektrostatisches Wechsel-und/oder Gleichfeld überlagert ist.

Fig. 1

Fig. 2

Fig. 3

Fig. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 239 098 (SCHMIDT)<br>* Spalte 4, Zeilen 17-20,26-45; Spalte 5, Zeilen 1-8,44-50; Anspruch 1; Figuren 1-5 * | 1,4,5,7 ,13 | A 47 C 23/00<br>A 47 C 27/00<br>A 61 N 1/42 |
| Y | | 14,17-21 | |
| X | DE-U-8 714 410 (BUSCHKY)<br>* Seite 4, Zeilen 2-7,17-22; Seite 6, Zeilen 15-22; Figur 1 * | 1,4-6 | |
| X | EP-A-0 048 451 (DROLET)<br>* Zusammenfassung; Seite 13, Zeilen 14-29; Seite 14, Zeilen 7-19; Seite 28, Zeile 19 - Seite 29, Zeile 18; Seite 34, Zeile 27 - Seite 35, Zeilen 3,11-14; Figuren 1,7,14,16 * | 1-5,7,9 -12,16 | |
| A | | 15 | |
| Y | EP-A-0 137 072 (ASAHI INDUSTRY CO., LTD)<br>* Seite 2, Zeile 5 von unten - Seite 4, Zeile 3 von unten; Figuren 1-5 * | 14,17 | RECHERCHIERTE SACHGEBIETE (Int. Cl.4) |
| A | | 13 | A 47 C<br>A 61 N |
| Y | DE-A-3 541 352 (PLANETA HAUSGERÄTE GmbH & CO., ELEKTROTECHNIK KG)<br>* Ansprüche 1,3,4 * | 18,19 | |
| D,Y | EP-A-0 144 610 (BOHN)<br>* Seite 1, Zeile 22 - Seite 2, Zeile 6; Seite 3, Zeilen 12-18 * | 20,21 | |
| A | DE-A-3 507 777 (WAGNER)<br>--- -/- | | |

Der vorliegende Recherchenbericht wurde für alle Patentansprüche erstellt

| Recherchenort | Abschlußdatum der Recherche | Prüfer |
|---|---|---|
| DEN HAAG | 31-05-1989 | DE COENE P.J.S. |

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 209 246 (DAW) --- | | |
| A | EP-A-0 099 734 (TESLA) ----- | | |

RECHERCHIERTE SACHGEBIETE (Int. Cl.4)

Der vorliegende Recherchenbericht wurde für alle Patentansprüche erstellt

| Recherchenort | Abschlußdatum der Recherche | Prüfer |
|---|---|---|
| DEN HAAG | 31-05-1989 | DE COENE P.J.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
................................................................
& : Mitglied der gleichen Patentfamilie, übereinstimmendes Dokument

EPO FORM 1503 03.82 (P0403)